# EUROPÄISCHE PATENTSCHRIFT

(11) **EP 1 503 704 B1**
(45) Veröffentlichungstag und Bekanntmachung des Hinweises auf die Patenterteilung: **11.06.2008**
(21) Anmeldenummer: 03732347.4
(22) Anmeldetag: 12.05.2003
(51) Int. Cl.: A61F 2/38

(54) **TIBIAKOMPONENTE UND GLEITPLATTE EINER KNIEGELENKENDOPROTHESE**
TIBIA COMPONENT AND SLIDING PLATE OF A KNEE-JOINT ENDOPROTHESIS
ELEMENT DE TIBIA ET PLAQUE DIRECTRICE APPARTENANT A UNE ENDOPROTHESE D'ARTICULATION DE GENOU

(30) Priorität: 14.05.2002 DE 10221272
(43) Veröffentlichungstag der Anmeldung: 09.02.2005
(73) Patentinhaber: Brehm, Peter, 91085 Weisendorf (DE)
(72) Erfinder: Brehm, Peter, 91085 Weisendorf (DE)
(74) Vertreter: Schneck, Herbert
(86) Internationale Anmeldenummer: PCT/EP2003/004936
(87) Internationale Veröffentlichungsnummer: WO 2003/094804

(56) Entgegenhaltungen:
- EP-A- 0 838 204
- EP-A- 0 904 748
- FR-A- 2 748 389
- US-A- 5 489 311
- US-A- 6 090 144

## Beschreibung

Die Erfindung richtet sich auf eine Tibiakomponente gemäß dem Oberbegriff von Anspruch 1.

Eine derartige Kniegelenkendoprothese ist beispielsweise aus US-A-6090144 bekannt.

Grundsätzlich ist es Ziel eines Kniegelenkersatzes, durch eine Endoprothese die Funktion des Gelenkes soweit wie möglich wieder herzustellen. Dabei müssen besonders Kinematik und Biomechanik mit ihrem Prinzip der Roll-Gleit-Drehbewegung berücksichtigt werden. Eine Kniegelenkprothese muss einen solchen Bewegungsvorgang nachvollziehen und die Beanspruchung durch Kippung, Schub und Drehung ohne Schaden für das Knochenlager und für den Kapselbandapparat auffangen können.

Hierzu sind einerseits Endoprothesen bekannt, bei welchen Tibia-Plateau-Teil und Femurkomponente gekoppelt sind sowie Endoprothesen der gattungsgemäßen Art, bei welchen eine solche Kopplung nicht vorgesehen ist. Bei solchen bikondylären Prothesen befinden sich Konzepte im Einsatz, die eine Wahl unterschiedlicher Freiheitsgrade in der relativen Bewegung der Polyethylen-Gleitkomponenten zu den Tibiakomponenten erlauben. Sie bestehen in starr fixierten Gleitplatten, rotierenden bzw. rotierenden mit einem translotorischen Freiheitsgrad (anterior-posterior) und frei schwimmenden Plattformen. Unter den heutigen Bedingungen im Operationssaal besteht in einer reduzierten Anzahl von Instrumenten und Implantaten und einer möglichst freien Umsetzbarkeit intraoperativer Entscheidungen ein wesentlicher Wettbewerbsvorteil, wobei zu diesen Entscheidungen der individuell beste Kompromiss zwischen Mobilität und Stabilität gehört. Eine feste Polyethylen-Gleitplatte ergibt die höchste Gelenkstabilität, eine frei schwimmende, bevorzugt bei stabiler Bandsituation eingesetzt, lässt die höchste Mobilität zu.

Bei der Formgebung der bikondylären Femur- und Gleitkomponente muss berücksichtigt werden, dass mediale und laterale Artikulationsflächen hoher Kongruenz zwar die Druckbelastung auf das Polyethylen durch eine vergrößerte Auflagefläche reduzieren, jedoch die Freiheitsgrade der natürlichen Roll- Gleitbewegung des Gelenkes mit axialer Tibiarotation einschränken können, was bei einer fixen Gleitkomponente zu hohen Scher-und Torsionskräften führt. Unerwünschte Folgen sind neben frühzeitiger Implantatlockerung vor allem hohe lokale Beanspruchung, Deformationen und Verschließ der Polyethylen-Gleitkomponente.

Der Erfindung liegt deshalb die Aufgabe zu Grunde, ein modulares Konzept zu realisieren, wobei untereinander kompatible Polyethylen-Gleitplatten mit unterschiedlichen Mobilitätsgraden auf ein und dasselbe Tibia-Plateau aufsetzbar sein sollen, so dass sich die Anzahl der im Operationssaal vorzuhaltenden Tibia-Plateau-Teile wesentlich reduzieren lässt. Darüber hinaus soll der Vorteil einer hochkongruenten Passform von Femur- und Gleitkomponente genutzt werden, aber eine Reduzierung der bei dieser Paarung auftretenden Rotationskräfte erreicht werden.

Diese Aufgabe wird erfindungsgemäß gelöst gemäß dem kennzeichnenden Teil von Anspruch 1.

Gegenüber der herkömmlichen Rotationssicherung unmittelbar auf der Tibiakomponente usw. bewirkt die distale Verlagerung um einige Zentimeter, wie sie durch die erfindungsgemäße Ausgestaltung erreicht wird, eine solche Übertragung der auftretenden Rotationsbewegung auf die Polyethylen-Gleitplatte, dass lokal auftretende Spitzenbelastungen mit hohem Polyethylenverschleiß vermieden werden und doch gleichzeitig unterschiedliche Gleitplatten mit dementsprechend unterschiedlichen Mobilitätsgraden sich in einfachster Weise intraoperativ einsetzen lassen.

Durch die Kompatibilität dieser Gleitplatten mit dem Tibia-Plateau-Teil, insbesondere der Tragplatte und dem Verankerungsabschnitt ermöglicht die erfindungsgemäße Ausgestaltung einen modularen Aufbau, der zahlreiche Vorteile mit sich bringt:

Wie bereits erwähnt, sind neben einer reduzierten Anzahl von Implantaten und Instrumenten für die klinische Akzeptanz von Kniegelenksystemen die intraoperativen Entscheidungsfreiheiten und die intraoperativen Rückzugsmöglichkeiten von entscheidender Bedeutung. Die Auswahl und das Einsetzen der Gleitplatte zählt zu den letzten Schritten der OP vor Nahtbeginn. Hierbei wird die Funktion des kompletten Kniegelenks in Beugung und Streckung vor allem hinsichtlich der durch die Bandsituation gegebenen Gelenkstabilität geprüft. Die erfindungsgemäße Ausgestaltung ermöglicht in dieser Phase eine Differenzierung der Gelenkausgestaltungen in fix, mobil rotierend, mobil anterior-posterior und rotierend (auch multidirektional) sowie mobil schwimmend und rotierend.

Beispielsweise ist folgende typische operative Situation vorstellbar:

Femur- und Tibiakomponente sind fest (zementiert oder zementfrei) implantiert. Das erfindungsgemäße Implantatsystem bietet dem Operateur die Möglichkeit, immer noch frei zu entscheiden, welche Gleitplattenvariante er einsetzen will. Dies wird ermöglicht durch die distale Verlagerung der Rotationssicherung, wie sie nach der Erfindung vorgesehen ist. Reduzierung der Operationsdauer, Schonung des knöchernen Implantatlagers und der Knochenkontaktfläche sowie Einsparung einer nicht mehr verwendbaren Tibiakomponente sind die klaren Vorteile. Bei Systemen nach dem Stand der Technik muss dem gegenüber bisher zum Wechseln von einer mobilen zu einer fixen Gleitplatte oder umgekehrt auch die Tibiakomponente mit ausgewechselt werden.

Ein weiterer wesentlicher Vorteil besteht gemäß der Erfindung darin, dass bei einer Revision die festen Femur- und Tibiakomponenten im Knochen belassen werden können und lediglich die Gleitplatte, abgestimmt auf die aktuelle Bandsituation, muss ausgewechselt werden. Der nach dem Stand der Technik bisher erforderliche Ausbau einer festeingewachsenen Tibiakomponente ist mit einem hohen Verlust an gesundem Knochen verbunden.

Die nicht-kreisförmige Ausnehmung weist vorzugsweise eine annähernd dreieckige Grundform mit abgerundeten Ecken auf, wodurch Spitzenbelastungen vermieden und eine zuverlässige Drehsicherung erreicht werden.

Bei einer weiteren Ausführungsform kann auch vorgesehen sein, dass der Verankerungsabschnitt der Tibiakomponente im Inneren eine nicht-kreisförmige positive Oberflächenkontur aufweist, die in eine nicht-kreisförmige negative Oberflächenkontur im Ansatz der Gleitplatte eingreift.

Eine weitere Ausführungsform sieht eine formschlüssig-drehfeste Verbindung vor, bei der die jeweils positiven und negativen Oberflächenkonturen der Gleitplatte und des Tibia-Plateau-Teils exzentrisch zur Ausnehmung und dem Ansatz angeordnet sind. Bei dieser Ausführungsform sind sowohl kreisförmige als auch nicht-kreisförmige formschlüssig ineinandergreifende Oberflächenkonturen möglich.

Bei allen Ausführungsformen können axial zum Ansatz der Gleitplatte verlaufende metallische Versteifungen vorgesehen sein, durch die ein Abscheren des Polyethylens vermieden wird. Die metallischen Verstärkungen können einen kreisförmigen oder nicht-kreisförmigen Querschnitt aufweisen.

Nachfolgend wird die Erfindung anhand eines bevorzugten Ausführungsbeispiels in Verbindung mit der Zeichnung näher erläutert. Dabei zeigen:
- Fig. 1: eine Ansicht der Polyethylen-Gleitplatte mit Verankerungsabschnitt von unten,
- Fig. 2: eine Seitenansicht der Gleitplatte mit Verankerungsabschnitt,
- Fig. 3: eine Seitenansicht des Tibia-Plateau-Teils,
- Fig. 4: einen Längsschnitt durch das Tibia-Plateau-Teil und
- Fig. 5: eine Aufsicht auf das Tibia-Plateau-Teil im implantierten Zustand.

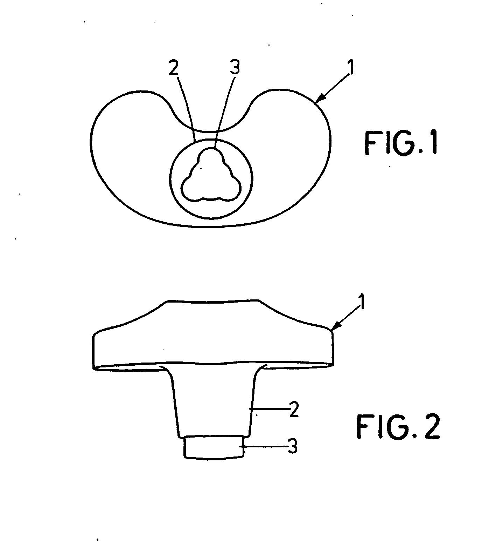

Eine in Fig. 1 und 2 dargestellte Gleitplatte 1 aus Polyethylen weist eine nierenförmige Grundform auf, wobei sich ein Ansatz 2 nach unten weg erstreckt, der einen Endabschnitt 3 aufweist, welcher eine, in Fig. 1 erkennbare, dreieckige Grundform mit abgerundeten Ecken aufweist.

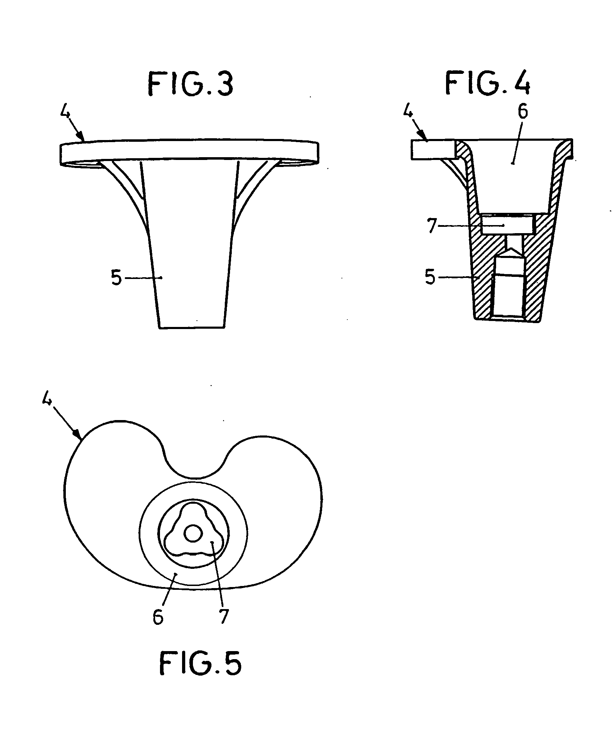

Die in Fig. 3 bis 5 dargestellte Tragplatte 4 ist mit einem sich nach unten erstreckenden Verankerungsabschnitt 5 versehen, der eine Ausnehmung 6 aufweist, welche der Form des Ansatzes 2 der Gleitplatte 1 entspricht.

An der Unterseite der Ausnehmung 6 ist eine Ausnehmung 7 kleineren Durchmessers vorgesehen, welche eine dreieckige Grundform mit abgerundeten Ecken aufweist und in ihrer Außenkontur der Außenkontur des Endabschnitts 3 entspricht.

Wird dementsprechend die in Fig. 1 und 2 dargestellte Gleitplatte 1 mit dem Ansatz 2 voraus in die Ausnehmung 6 eingeführt, greift der Endabschnitt 3 formschlüssig in die Ausnehmung 7 ein, sodass eine drehfeste Verbindung hergestellt wird.

Dementsprechend ist es möglich, unter Verwendung ein und desselben Tibia-Plateau-Teils 4 Gleitplatten 1 mit unterschiedlichem Mobilitätsgrad zu verwenden.

## Patentansprüche

1. Tibiakomponente mit einem Verankerungsabschnitt zur Festlegung im Knochen sowie einer Tragplatte für eine Gleitplatte aus Polyethylen, wobei die Tibiakomponente zusammenwirkt mit einer Femurkomponente, welche mit gekrümmten Artikulationsflächen auf der Gleitplatte aufliegt, wobei die Gleitplatte (1) einen Ansatz (2) aufweist, der sich in das Innere des Verankerungsabschnitts (5) des Tibia-Plateau-Teils (4) erstreckt und distal vom Tibia-Plateau-Teil (4) formschlüssig-drehfest mit dem Verankerungsabschnitt (5) verbunden ist, **dadurch gekennzeichnet, dass** der Verankerungsabschnitt (5) eine distale Ausnehmung (7) mit einem nicht-kreisförmigen Querschnitt und der Ansatz (2) der Gleitplatte (1) einen Endabschnitt (3) mit entsprechender Außenkontur aufweist, wobei dieser Endabschnitt (3) formschlüssig in die nicht-kreisförmige Ausnehmung (7) eingreift.

2. Tibiakomponente und Gleitplatte nach Anspruch 1, **dadurch gekennzeichnet, dass** die nicht-kreisförmige Ausnehmung (7) eine annähernd dreieckige Grundform mit abgerundeten Ecken aufweist.

3. Tibiakomponente und Gleitplatte nach Anspruch 1, **dadurch gekennzeichnet, dass** die Gleitplatte (1) aus einem Polyethylen mit ultrahohem Molekulargewicht und/oder aus kreuzvernetztem Polyethylen besteht.

## Claims

1. A tibial component comprising an anchor portion for being fixed in bone and a tray for a sliding plate of polyethylene, the tibial component cooperating with a femoral component which rests on the sliding plate by curved articulation surfaces, wherein the sliding plate (1) has an attachment (2) which extends inside the anchor portion (5) of the tibial plateau component (4) and which is non-rotatably connected to the anchor portion (5) by positive fit distally of the tibial plateau component (4), **characterized in that** the anchor portion (5) comprises a distal recess (7) of noncircular cross-sectional shape; and **in that** the attachment (2) of the sliding plate (1) comprises an end portion (3) of corresponding outer contour, this end portion (3) engaging with the non-circular recess (7) by positive fit.

2. A tibial component and sliding plate according to claim 1, **characterized in that** the non-circular recess (7) has an approximately triangular basic shape with rounded corners.

3. A tibial component and sliding plate according to claim 1, **characterized in that** the sliding plate (1) consists of polyethylene of ultrahigh molecular weight and/or cross-linked polyethylene.

## Revendications

1. Élément de tibia comprenant une section d'ancrage pour une fixation dans l'os ainsi qu'une plaque-support pour une plaque de glissement en polyéthylène, l'élément de tibia coopérant avec un élément de fémur, lequel repose avec une surface d'articulation cintrée sur la plaque de glissement, la plaque de glissement (1) comprenant un appendice (2) qui se prolonge à l'intérieur de la section d'ancrage (5) de la partie de plateau tibial (4) et qui est relié distalement par complémentarité de forme de façon bloquée en rotation à la section d'ancrage (5) à partir de la partie de plateau tibial (4), **caractérisés en ce que** la section d'ancrage (5) comprend un évidement distal (7) avec une coupe transversale non circulaire et l'appendice (2) de la plaque de glissement (1) une section d'extrémité (3) avec un contour extérieur correspondant, la section d'extrémité (3) s'engageant par complémentarité de forme dans l'évidement (7) non circulaire.

2. Élément de tibia et plaque de glissement selon la revendication 1, **caractérisés en ce que** l'évidement non circulaire (7) comp-rend une forme de base à peu près triangulaire avec des angles arrondis.

3. Élément de tibia et plaque de glissement selon la revendication 1, **caractérisés en ce que** la plaque de glissement (1) en polyéthylène se compose d'un poids moléculaire très élevé et/ou de polyéthylène réticulé.
